# EUROPEAN PATENT SPECIFICATION

(11) **EP 3 735 946 B1**
(45) Date of publication and mention of the grant of the patent: **03.05.2023**
(21) Application number: 19173178.5
(22) Date of filing: 07.05.2019
(51) Int. Cl.: A61F 9/007, C03C 4/00

(54) **SHUNT FOR DRAINING OCULAR FLUID**
SHUNT ZUR ABLEITUNG VON AUGENFLÜSSIGKEIT
DÉRIVATION POUR DRAINER UN FLUIDE OCULAIRE

(43) Date of publication of application: 11.11.2020
(73) Proprietor: Taiwan Fiber Optics, Inc., 104 Zhongshan Dist. Taipei City (TW)
(72) Inventor: LU, Luke, San Diego, CA 92131 (US); TSENG, Hsiao Sen, 420 Taichung City (TW); LU, Michelle, San Diego, CA 92131 (US); LU, Emily, San Diego, CA 92131 (US)
(74) Representative: Lang, Christian

(56) References cited:
- EP-A1- 3 034 480
- WO-A1-2016/149425
- US-A1- 2013 325 024

## Description

### BACKGROUND OF THE INVENTION

### 1. Field of the Invention

The invention relates to tube shunts and more particularly to a shunt having a tubular body adapted to be attached to sclera for draining ocular fluid from the eye, thereby lowering ocular pressure.

### 2. Description of Related Art

For treating glaucoma, conventional shunts for draining ocular fluid are made of metal or polymer. The shunt may form a scab over a healed cut and the eye of a patient may feel a degree of discomfort due to heavy weight even the shunt is small. Further, there is a concern of risk for metal and polymer staying in the human body after some years, It is always a long term concern of risk, another surgery for removing the shunt is required. It is understood that there is risk in surgery.

Thus, the need for improvement of the shunt still exists.
Patent application publication US 2013/0325024 A1 discloses a shunt for draining ocular fluid having the features of claim 1. From patent application publication WO 2016/149425 A1 a shunt for draining ocular fluid is known having the features of claim 2.

### SUMMARY OF THE INVENTION

It is therefore one object of the invention to provide a shunt for draining ocular fluid, comprising a tubular body formed of a mesh material including bioactive glass fibers and collagen, the tubular body including an implantation member and a conduit through the implantation member; wherein the implantation member and the conduit are formed integrally.

It is another object of the invention to provide a shunt for draining ocular fluid, comprising a tubular body formed of a bioactive glass material, the tubular body including an implantation member and a conduit; wherein the implantation member and the conduit are formed integrally; wherein the implantation member includes at least one anchor member; and wherein the conduit passes through the implantation member.

The invention has the following advantages and benefits in comparison with the conventional art:
By constructed as above, the tubular body can be attached to sclera so that ocular fluid may leave the eye to flow through the conduit, thereby lowering ocular pressure. Further, the tubular body is formed of a bioactive glass material, i.e., capable of being absorbed by the body tissues. Specifically, the tubular body is attracted to the body tissues and tends to be dissolved by the body tissues. Thus, a glaucoma patient may have a minimum feeling of discomfort when the tubular body is attached to the sclera. Furthermore, the tubular body may be absorbed by the body tissues as time goes by, thereby forming a tubular, flexible member for healing purposes.

The tubular body may be constructed as a double-layer member in which a first layer of the tubular body is formed of the bioactive glass material and a second layer thereof is formed of a bio-glass material. Specifically, the second layer is formed of a bioactive glass material having a high composition of silica or a bio-inert glass material. Thus, it is possible of controlling strength of the tubular body and time for the tubular body to be absorbed by the body tissues by adjusting weight percentages of compositions of the first and second layers.

The above and other objects, features and advantages of the invention will become apparent from the following detailed description taken with the accompanying drawings.

### BRIEF DESCRIPTION OF THE DRAWINGS

FIG. 1 is a perspective view of a shunt for draining ocular fluid according to a first preferred embodiment of the invention;
FIG. 2 is a perspective view of a shunt for draining ocular fluid according to a second preferred embodiment of the invention;
FIG. 3 is another perspective view of FIG. 2 showing bottom characteristics of the shunt (i.e., the tubular body);
FIG. 4 is a longitudinal sectional view of the shunt shown in FIG. 2;
FIG. 4A is a view similar to FIG. 4 showing a double-layer configuration of the shunt;
FIG. 5 is a perspective view of a shunt for draining ocular fluid according to a third preferred embodiment of the invention;
FIG. 6 is a longitudinal sectional view of the shunt shown in FIG. 5;
FIG. 6A is a view similar to FIG. 6 showing a double-layer configuration of the shunt;
FIG. 7 is a perspective view of a shunt for draining ocular fluid according to a fourth preferred embodiment of the invention;
FIG. 7A is a longitudinal sectional view of the shunt shown in FIG. 7;
FIG. 8 is a perspective view of a shunt for draining ocular fluid according to a fifth preferred embodiment of the invention;
FIG. 8A is a side elevation of FIG. 8;
FIG. 9 schematically depicts inserting an implantation device through an anterior section of an eye to attach the tubular body of the first preferred embodiment to sclera;
FIG. 10 is a view similar to FIG. 9 showing after the implantation device removal, the tubular body being ready to drain ocular fluid;
FIG. 11 is a detailed view of the area in a circle of FIG. 10; and
FIG. 12 is a view similar to FIG. 11 showing the tubular body of the third preferred embodiment attached to the sclera and draining ocular fluid.

### DETAILED DESCRIPTION OF THE INVENTION

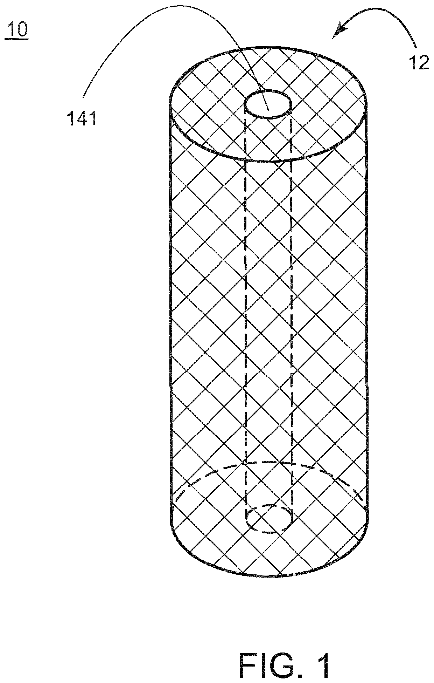

Referring to FIG. 1, a shunt for draining ocular fluid in accordance with a first preferred embodiment of the invention comprises a tubular body 10 formed of a mesh material including bioactive glass fiber and collagen. The tubular body 10 includes an implantation member 12 and a conduit 141 through the implantation member 12. The implantation member 12 and the conduit 141 are formed integrally. The dispersion capability of the tubular body 10 can be increased or decreased by different compositions of bioactive glass fibers, bioresorable fibers and collagen. The tubular body 10 allows ocular fluid in an anterior section of an eye to flow through in a controlled manner for lowering intraocular pressure. As shown, the tubular body 10 does not have a specific direction for implantation into the eye. Thus, the implantation member 12 can be disposed at either end of the tubular body 10.

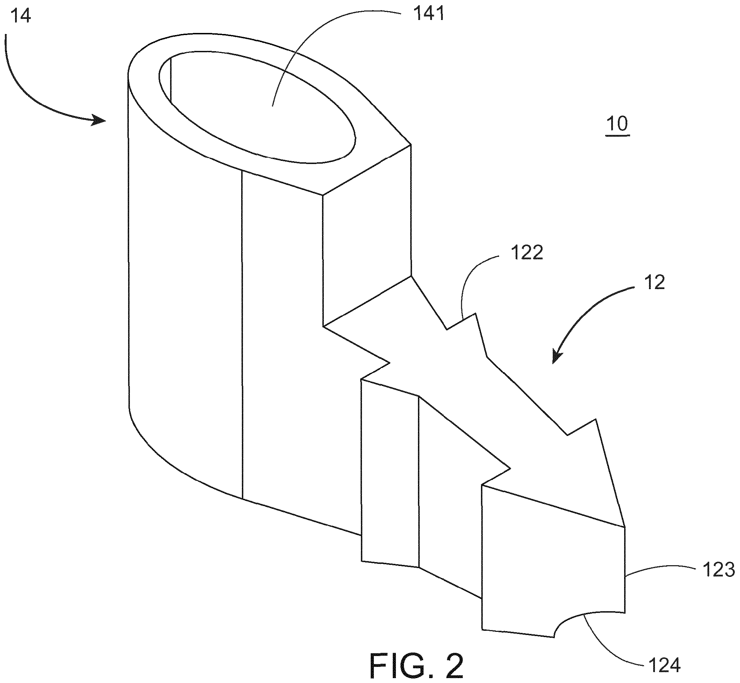

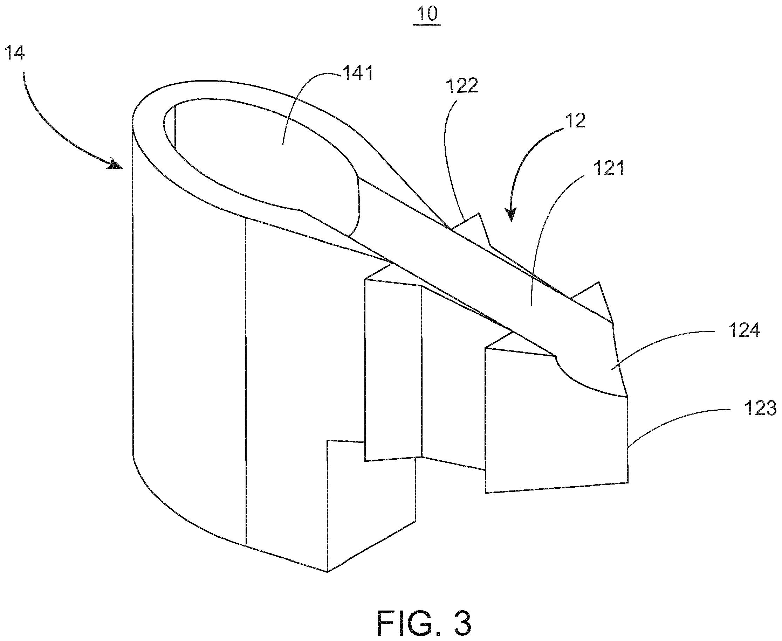

Referring to FIGS. 2 to 4, a shunt for draining ocular fluid in accordance with a second preferred embodiment of the invention comprises a tubular body 10 formed of a bioactive glass material. The tubular body 10 includes an implantation member 12 and a fluid guide member 14 extending out of the implantation member 12. A conduit 141 is disposed through the fluid guide member 14. The fluid guide member 14 is perpendicular to the implantation member 12 so that the tubular body 10 is shaped as an L in a longitudinal section. The implantation member 12 includes a sharp end 123, a cavity 124 on a lower portion of the sharp end 123, a plurality of sharp projections 122 on both sides of the implantation member 12, and a bottom channel 121 communicating with both the conduit 141 and the cavity 124. The conduit 141 has an oval opening at one end. It is noted that the opening may be shaped as a circle, a hexagon, or a polygon in other embodiments. The conduit 141 has a smooth surface and has an axial length of less than 0.5mm and a diameter of less than 0.2mm. By constructed as above, the tubular body 10 can be absorbed or be degraded in body tissues in several months to form a tubular, flexible member for healing purposes.

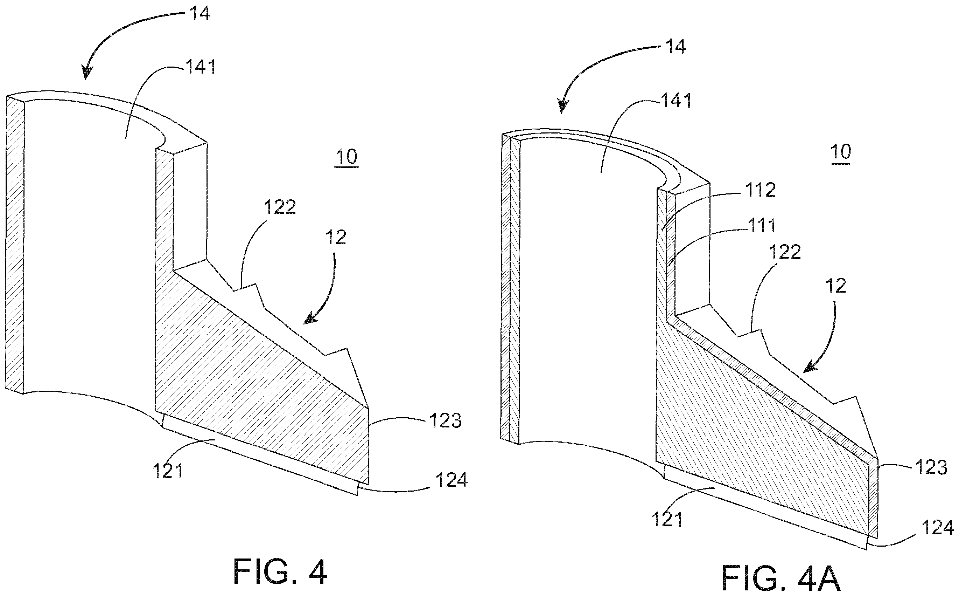

Referring to FIG. 4A, the tubular body 10 of the second preferred embodiment is constructed as a double-layer member in which a first layer 111 of the tubular body 10 is formed of the bioactive glass material and a second layer 112 thereof is formed of a bio-glass material. It may take several months for the second layer 112 to be absorbed or to be degraded in the body tissues if it is formed of a bioactive glass material having a high composition of silica. Alternatively, the second layer 112 is formed of a bio-inert glass material and the second layer 112 is not subject to be absorbed or to be degraded, i.e., remained in the body tissues forever.

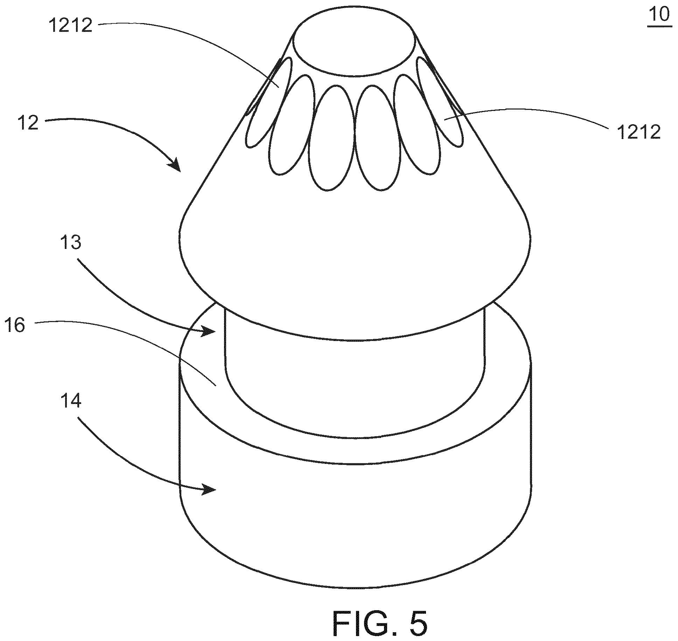

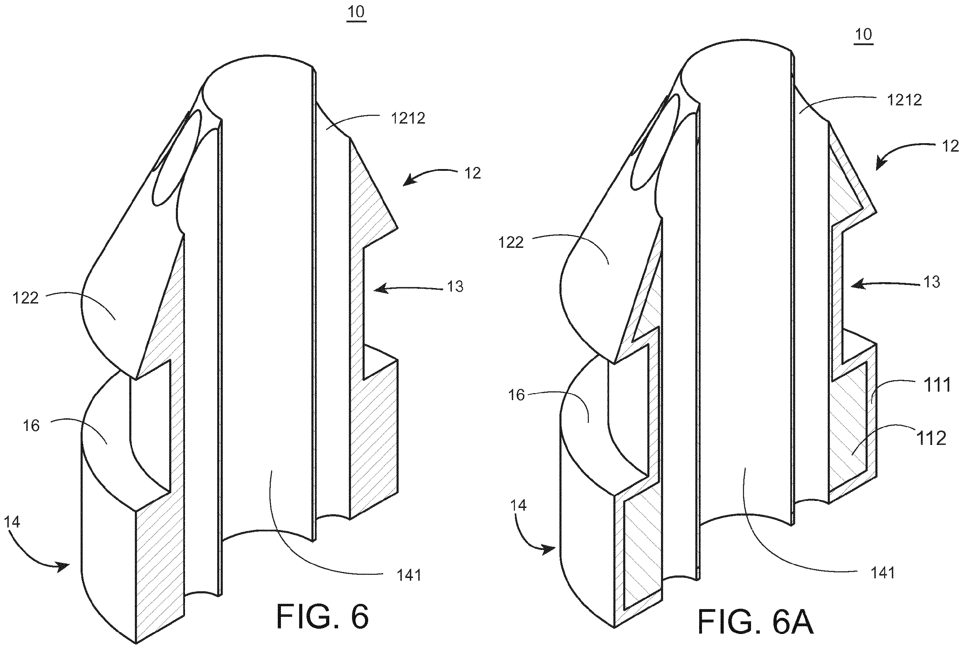

Referring to FIGS. 5 and 6, a shunt for draining ocular fluid in accordance with a third preferred embodiment of the invention comprises a tubular body 10 formed of a bioactive glass material. The tubular body 10 includes an implantation member 12 and a fluid guide member 14 extending out of the implantation member 12. A conduit 141 is disposed through the fluid guide member 14. The fluid guide member 14 is aligned with the implantation member 12 so that the tubular body 10 is shaped as an I in a side elevation. The implantation member 12 includes a tapered anchor member 122 at one end, a plurality of lengthwise tunnels 1212 equally spaced around and separated from the conduit 141, each tunnel 1212 being parallel to the conduit 141 and passing through the tubular body 10 to have two ends open, an annular groove 13 disposed between the tapered anchor member 122 and the fluid guide member 14, and an annular shoulder 16 disposed between the annular groove 13 and the fluid guide member 14. The conduit 141 has a circular opening at one end. It is noted that the opening may be shaped as an oval, a hexagon, or a polygon in other embodiments.

Referring to FIG. 6A, the tubular body 10 of the third preferred embodiment is constructed as a double-layer member in which an first layer 111 of the tubular body 10 is formed of the bioactive glass material and an second layer 112 thereof is formed of a bio-glass material. The second layer 112 may be formed of a bioactive glass material having a high composition of silica or a bio-inert glass material. It may take several years for the second layer 112 to be absorbed or to be degraded in the body tissues if it is formed of a bioactive glass material having a high composition of silica. Alternatively, the second layer 112 is formed of a bio-inert glass material and the second layer 112 is not subject to be absorbed or to be degraded, i.e., remained in the body tissues forever.

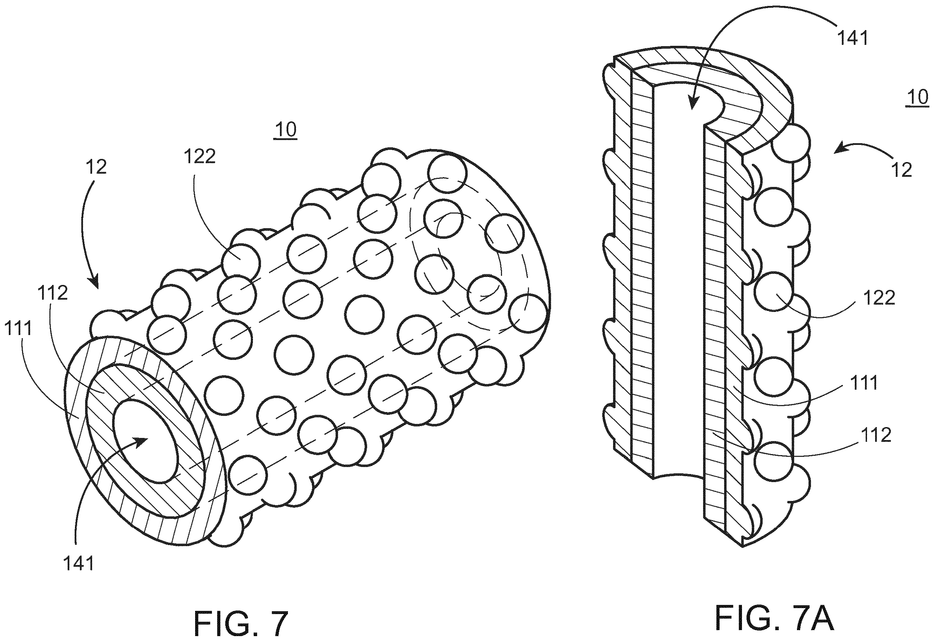

Referring to FIGS. 7 and 7A, a shunt for draining ocular fluid in accordance with a fourth preferred embodiment of the invention comprises a tubular body 10 including a first layer 111 formed of a bioactive glass material and a second layer 112 formed of a bio-glass material. The second layer 112 and the first layer 111 are fused by fusion process. Specifically, the second layer 112 is formed of a bioactive glass material having a high composition of silica or a bio-inert glass material. It may take several months for the second layer 112 to absorb or degrad in the body tissues if it is formed of a bioactive glass material having a high composition of silica. Alternatively, the second layer 112 is formed of a bio-inert glass material and the second layer 112 is not subject to degradation, i.e., remained in the body tissues forever.

A tubular implantation member 12 is comprised of the second layer 112 and the first layer 111 and includes a plurality of substantially half-spherical protrusions 122 on an outer surface for adhering purposes. A conduit 141 is integrally formed with and passes through the implantation member 12 to have two ends open.

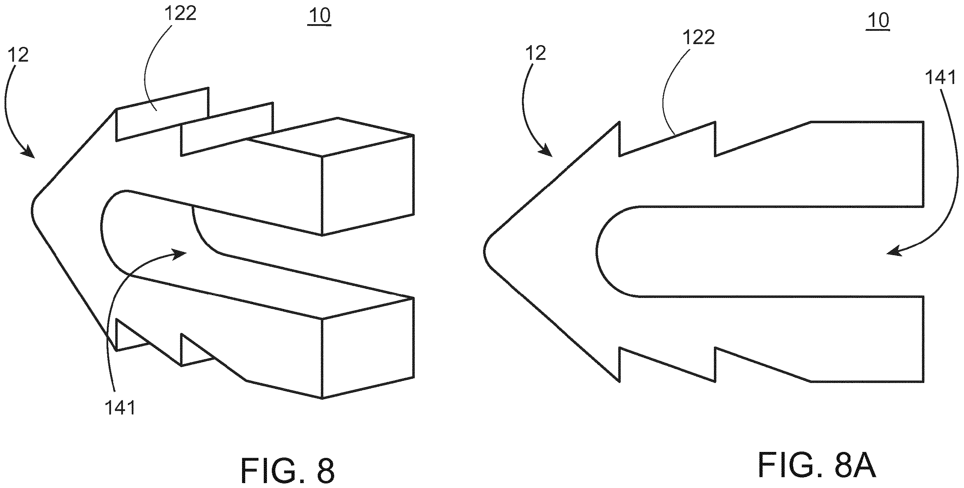

Referring to FIGS. 8 and 8A, a shunt for draining ocular fluid in accordance with a fifth preferred embodiment of the invention comprises a tubular body 10 including an implantation member 12 and a conduit 141 integrally formed with the implantation member 12. The implantation member 12 includes a plurality of sharp projections 122 on both sides. The conduit 141 is in the implantation member 12 and has one end and two sides open. The tubular body 10 is formed of a bioactive glass material. Thus the tubular body 10 can be absorbed or degraded in body tissues in several weeks. By constructed as above, the tubular body 10 can be attached to sclera so that ocular fluid may leave the eye to flow through the conduit 141, thereby lowering ocular pressure.

The tubular body 10 is formed of a bioactive glass material, i.e., capable of being absorbed by the body tissues. Specifically, the tubular body 10 is attracted to the body tissues and tends to be dissolved by the body tissues. Thus, a glaucoma patient may have a minimum feeling of discomfort when the tubular body 10 is attached to sclera. Also, the tubular body 10 may be absorbed by the body tissues as time goes by, thereby forming a tubular, flexible member for healing purposes. As an end, another surgery to remove the tubular body 10 from the sclera is not required.

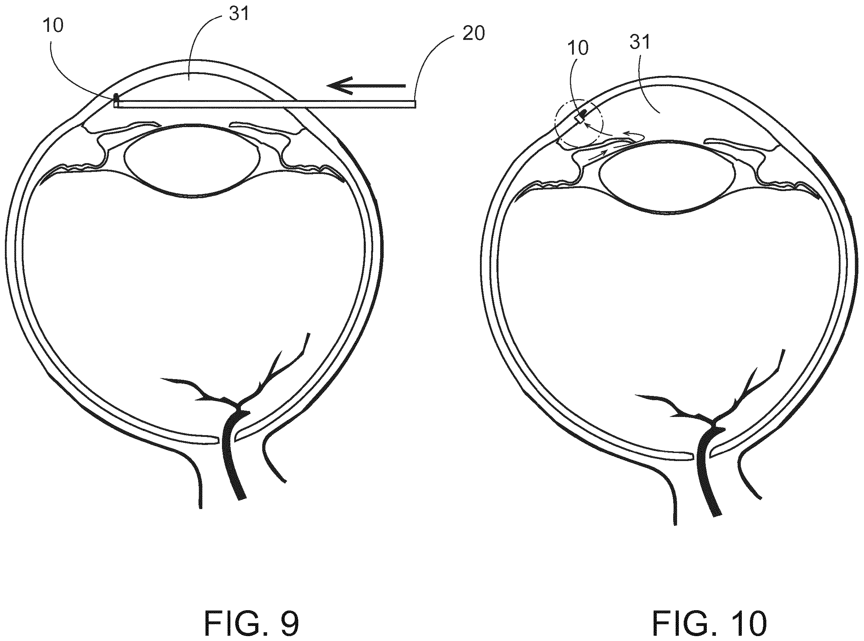

Referring to FIGS. 9 to 12, a number of implementations of the tubular body 10 are illustrated.

As shown in FIGS. 9 to 11 in conjunction with FIGS. 2 to 4A, the tubular body 10 is mounted at an end of an implantation device 20. The implantation device 20 passes through an anterior section 31 of an eye. The sharp end 123 of the implantation member 12 facilitates the tubular body 10 to enter and attach to the sclera. After attaching to the sclera, a medically employee may slightly adjust an angle of the implantation device 20 to attach a maximum portion of the implantation member 12 to the sclera.

In the second preferred embodiment, the implantation member 12 of the tubular body 10 is attached to the sclera, and the conduit 141 communicates with the anterior section 31. Thus, ocular fluid may leave the anterior section 31 through the conduit 141, thereby lowering intraocular pressure.

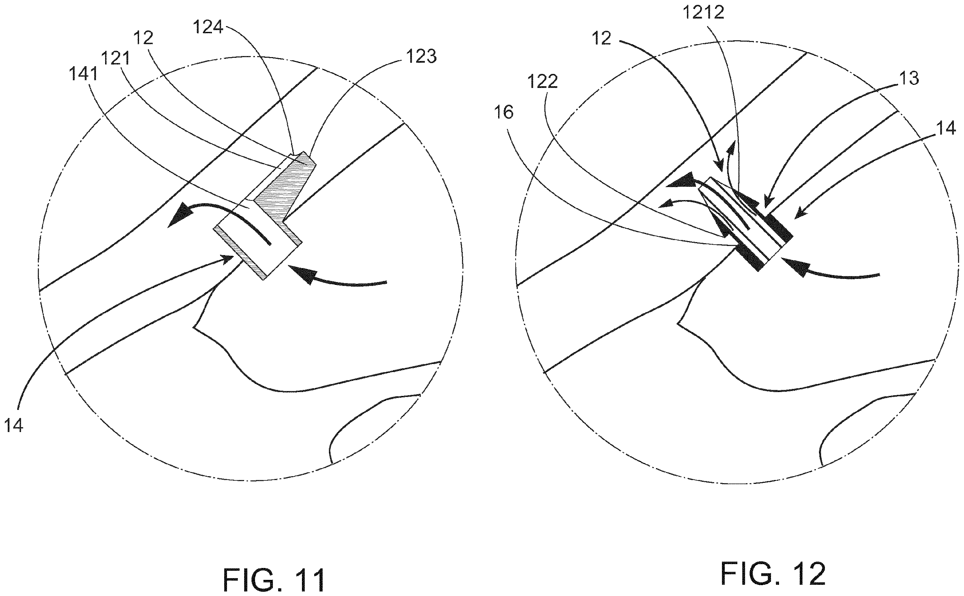

As shown in FIG. 12 in conjunction with FIGS. 5 to 6A, the implantation member 12 of the tubular body 10 of the third preferred embodiment is attached to the sclera, and the conduit 141 communicates with the anterior section 31. Thus, ocular fluid may leave the anterior section 31 through the conduit 141 or the tunnels 1212, thereby lowering intraocular pressure.

While the invention has been described in terms of preferred embodiments, those skilled in the art will recognize that the invention can be practiced with modifications within the scope of the appended claims.

## Claims

1. A shunt for draining ocular fluid, comprising:
a tubular body (10) including an implantation member (12) and a conduit (141) through the implantation member (12);
wherein the implantation member (12) and the conduit (141) are formed integrally
**characterised in that**
the tubular body (10) is formed of a mesh material including bioactive glass fibers that are capable of being absorbed by body tissue, bioresorable fibers and collagen.

2. A shunt for draining ocular fluid, comprising:
a tubular body (10) including an implantation member (12) and a conduit (141);
wherein the implantation member (12) and the conduit (141) are formed integrally;
wherein the implantation member (12) includes at least one anchor member (122); and
wherein the conduit (141) passes through the implantation member (12)
**characterised in that**
the tubular body (10) is formed of a bioactive glass material that is capable of being absorbed by body tissue.

3. The shunt for draining ocular fluid of claim 2, wherein the tubular body (10) includes a second layer (112) formed of either a bioactive glass material having a high composition of silica or a bio-inert glass material.

4. The shunt for draining ocular fluid of claim 2, wherein the tubular body (10) further comprises a fluid guide member (14) extending out of the implantation member (12); wherein the fluid guide member (14) is perpendicular to the implantation member (12) so that the tubular body (10) is shaped as an L in a longitudinal section; wherein the implantation member (12) includes a sharp end (123), a cavity on a lower portion of the sharp end (123), a plurality of sharp projections (122) on both sides of the implantation member (12), and a bottom channel (121) communicating with both the conduit (141) and the cavity (124).

5. The shunt for draining ocular fluid of claim 2, wherein the tubular body (10) further comprises a fluid guide member (14) extending out of the implantation member (12); wherein the fluid guide member (14) is aligned with the implantation member (12) so that the tubular body (10) is shaped as an I in a side elevation; and wherein the implantation member (12) includes a tapered anchor member (122) at one end, a plurality of lengthwise tunnels (1212) equally spaced around and separated from the conduit (141), each tunnel (1212) being parallel to the conduit (141) and passing through the tubular body (10), an annular groove (13) disposed between the tapered anchor member (122) and the fluid guide member (14), and an annular shoulder (16) disposed between the annular groove (13) and the fluid guide member (14).

6. The shunt for draining ocular fluid of claim 2, wherein the conduit (141) has two open sides.

7. The shunt for draining ocular fluid of claim 2, wherein each of the at least one anchor member (122) is substantially half-spherical or tapered.

8. The shunt for draining ocular fluid of claim 2, wherein the conduit (141) has an opening shaped as a circle, an oval, a hexagon, or a polygon.

## Patentansprüche

1. Shunt zur Drainage von Augenflüssigkeit, umfassend:
einen röhrenförmigen Körper (10) mit einem Implantationselement (12) und einer Leitung (141) durch das Implantationselement (12),
wobei das Implantationselement (12) und die Leitung (141) einstückig ausgebildet sind,
**dadurch gekennzeichnet, dass**
der röhrenförmige Körper (10) aus einem Netzmaterial gebildet ist, das bioaktive Glasfasern, die von Körpergewebe absorbiert werden können, bioresorbierbare Fasern und Kollagen enthält.

2. Shunt zur Drainage von Augenflüssigkeit, umfassend:
einen röhrenförmigen Körper (10) mit einem Implantationselement (12) und einer Leitung (141),
wobei das Implantationselement (12) und die Leitung (141) einstückig ausgebildet sind,
wobei das Implantationselement (12) mindestens ein Verankerungselement (122) aufweist, und
wobei die Leitung (141) durch das Implantationselement (12) verläuft,
**dadurch gekennzeichnet, dass**
der röhrenförmige Körper (10) aus einem bioaktiven Glasmaterial gebildet ist, das von Körpergewebe absorbiert werden kann.

3. Shunt zur Drainage von Augenflüssigkeit nach Anspruch 2, wobei der röhrenförmige Körper (10) eine zweite Schicht (112) enthält, die entweder aus einem bioaktiven Glasmaterial mit einem hohen Anteil an Siliziumdioxid oder aus einem bioinerten Glasmaterial besteht.

4. Shunt zur Drainage von Augenflüssigkeit nach Anspruch 2, wobei der röhrenförmige Körper (10) ferner ein Flüssigkeitsführungselement (14) umfasst, das sich aus dem Implantationselement (12) heraus erstreckt, wobei das Flüssigkeitsführungselement (14) senkrecht zu dem Implantationselement (12) steht, so dass der röhrenförmige Körper (10) in einem Längsschnitt die Form eines L aufweist, wobei das Implantationselement (12) ein spitzes Ende (123), einen Hohlraum an einem unteren Abschnitt des spitzen Endes (123), eine Vielzahl von spitzen Vorsprüngen (122) auf beiden Seiten des Implantationselements (12) und einen Bodenkanal (121), der sowohl mit der Leitung (141) als auch mit dem Hohlraum (124) in Verbindung steht, aufweist.

5. Shunt zum Ableiten von Augenflüssigkeit nach Anspruch 2, wobei der röhrenförmigen Körper (10) ferner ein Flüssigkeitsführungselement (14) umfasst, das sich aus dem Implantationselement (12) heraus erstreckt, wobei das Flüssigkeitsführungselement (14) mit dem Implantationselement (12) ausgerichtet ist, so dass der röhrenförmige Körper (10) in einer Seitenansicht die Form eines I hat, und wobei das Implantationselement (12) ein sich verjüngendes Verankerungselement (122) an einem Ende, eine Vielzahl von längsgerichteten Tunneln (1212), die gleichmä-ßig um die Leitung (141) herum beabstandet und von dieser getrennt sind, wobei jeder Tunnel (1212) parallel zu der Leitung (141) ist und durch den röhrenförmigen Körper (10) verläuft, eine ringförmige Nut (13), die zwischen dem sich verjüngenden Verankerungselement (122) und dem Flüssigkeitsführungselement (14) angeordnet ist, und eine ringförmige Schulter (16), die zwischen der ringförmigen Nut (13) und dem Flüssigkeitsführungselement (14) angeordnet ist, aufweist.

6. Shunt zum Ableiten von Augenflüssigkeit nach Anspruch 2, wobei die Leitung (141) zwei offene Seiten aufweist.

7. Shunt zur Drainage von Augenflüssigkeit nach Anspruch 2, wobei jedes der mindestens einen Verankerungselemente (122) im Wesentlichen halbkugelförmig oder konisch ist.

8. Shunt zur Drainage von Augenflüssigkeit nach Anspruch 2, wobei die Leitung (141) eine Öffnung in Form eines Kreises, eines Ovals, eines Sechsecks oder eines Polygons aufweist.

## Revendications

1. Shunt pour drainer un fluide oculaire, comprenant :
un corps tubulaire (10) incluant un élément d'implantation (12) et un conduit (141) à travers l'élément d'implantation (12) ;
dans lequel l'élément d'implantation (12) et le conduit (141) sont formés solidaires, **caractérisé en ce que**
le corps tubulaire (10) est constitué d'un matériau maillé incluant des fibres de verre bio-actives qui sont aptes à être absorbées par un tissu corporel, des fibres biorésorbables et du collagène.

2. Shunt pour drainer un fluide oculaire, comprenant :
un corps tubulaire (10) incluant un élément d'implantation (12) et un conduit (141) ;
dans lequel l'élément d'implantation (12) et le conduit (141) sont formés solidaires ;
dans lequel l'élément d'implantation (12) inclut au moins un élément d'ancrage (122) ; et
dans lequel le conduit (141) passe à travers l'élément d'implantation (12) **caractérisé en ce que**
le corps tubulaire (10) est constitué d'un matériau en verre bio-actif qui est apte à être absorbé par un tissu corporel.

3. Shunt pour drainer un fluide oculaire selon la revendication 2, dans lequel le corps tubulaire (10) inclut une seconde couche (112) constituée soit d'un matériau en verre bio-actif présentant une haute teneur en silice, soit d'un matériau en verre bio-inerte.

4. Shunt pour drainer un fluide oculaire selon la revendication 2, dans lequel le corps tubulaire (10) comprend en outre un élément de guidage de fluide (14) s'étendant hors de l'élément d'implantation (12) ; dans lequel l'élément de guidage de fluide (14) est perpendiculaire à l'élément d'implantation (12) de sorte que le corps tubulaire (10) est en forme de L dans une section longitudinale ; dans lequel l'élément d'implantation (12) inclut une extrémité pointue (123), une cavité sur une partie inférieure de l'extrémité pointue (123), une pluralité de saillies pointues (122) de part et d'autre de l'élément d'implantation (12), et un canal inférieur (121) communiquant à la fois avec le conduit (141) et la cavité (124).

5. Shunt pour drainer un fluide oculaire selon la revendication 2, dans lequel le corps tubulaire (10) comprend en outre un élément de guidage de fluide (14) s'étendant hors de l'élément d'implantation (12) ; dans lequel l'élément de guidage de fluide (14) est aligné sur l'élément d'implantation (12) de sorte que le corps tubulaire (10) est en forme de 1 sur une hauteur latérale ; et dans lequel l'élément d'implantation (12) inclut un élément d'ancrage conique (122) au niveau d'une extrémité, une pluralité de tunnels longitudinaux (1212) régulièrement espacés autour et séparés du conduit (141), chaque tunnel (1212) étant parallèle au conduit (141) et passant à travers le corps tubulaire (10), une rainure annulaire (13) disposée entre l'élément d'ancrage conique (122) et l'élément de guidage de fluide (14), et un épaulement annulaire (16) disposé entre la rainure annulaire (13) et l'élément de guidage de fluide (14).

6. Shunt pour drainer un fluide oculaire selon la revendication 2, dans lequel le conduit (141) présente deux côtés ouverts.

7. Shunt pour drainer un fluide oculaire selon la revendication 2, dans lequel chacun du au moins un élément d'ancrage (122) est sensiblement en forme de demi-sphère ou conique.

8. Shunt pour drainer un fluide oculaire selon la revendication 2, dans lequel le conduit (141) présente une ouverture en forme de cercle, d'ovale, d'hexagone, ou de polygone.
